Europäisches Patentamt

European Patent Office

Office européen des brevets

(11) Publication number: **0 328 702**

**A1**

# EUROPEAN PATENT APPLICATION

(21) Application number: **88102286.7**

(22) Date of filing: **17.02.88**

(51) Int. Cl.⁴: **A61L 9/04 , A61L 9/01**

(43) Date of publication of application:
**23.08.89 Bulletin 89/34**

(84) Designated Contracting States:
**DE ES FR GB NL**

(71) Applicant: **SYNT S.N.C. DI INVERNIZZI SILVANO E TOZZI DINA**
**Via Gagliani 5**
**I-40069 Zola Predosa (Bologna)(IT)**

(72) Inventor: **Invernizzi, Silvano**
**Via Gagliani 5**
**I-40069 Zola Predosa (Province Bologna)(IT)**

(74) Representative: **Modiano, Guido et al**
**MODIANO, JOSIF, PISANTY & STAUB**
**Modiano & Associati Via Meravigli, 16**
**I-20123 Milan(IT)**

(54) Deodorant composition and containment apparatus for enclosed spaces, in particular for cars, houses and the like.

(57) The deodorant composition for enclosed spaces, in particular for cars, houses and the like, is constituted by a gelatine comprising at least 50% by weight of an essential oil and less than 50% by weight of a thickening agent, preferably colloidal silica.

EP 0 328 702 A1

Xerox Copy Centre

# DEODORANT COMPOSITI0ON AND CONTAINMENT APPARATUS FOR ENCLOSED SPACES, IN PARTICU-LAR FOR CARS, HOUSES AND THE LIKE

The present invention relates to a deodorant composition and containment apparatus for small enclosed spaces, in particular for cars, houses and the like.

Various types of deodorant compositions are currently known, composed of essential oils emulsified with water to which suitable thickening substances are then added, in particular of the type of alginates. Gelatinous substances are obtained (hereinafter termed gelatines for brevity) in which the essence content which can be introduced is however modest, since the thickening substances do not dissolve in oil and therefore only the water is thickened. The essence is therefore dispersed in the aqueous carrier, significantly limiting the emanation intensity and duration of the deodorant itself.

The object of the present invention is to eliminate the above described problem by providing a deodorant composition for enclosed spaces, in particular for cars, houses and the like, which allows to concentrate in a limited quantity of gelatine large quantities of essential oil, so as to ensure a considerable and controllable emission intensity and duration.

This object is achieved, according to the invention, by a deodorant composition of controllable scent emission intensity for enclosed spaces, in particular for cars, houses and the like, consisting of a gelatine, comprising a mixture of at least 50% by weight of an essential oil with less than 50% of a thickening agent compatible with said oil.

Another object is to provide an apparatus for controlling the scent emission intensity of the deodorant composition.

The scent emission is controlled according to the invention by an apparatus, characterized in that it comprises an outer cylindrical shell having at least one lateral opening, and a container of said deodorant rotatably inserted in said outer shell and having a plurality of longitudinal slots adapted to face, more or less, according to the reciprocal angular position of said outer shell and said container, said opening so as to regulate release of said deodorant in enclosed spaces.

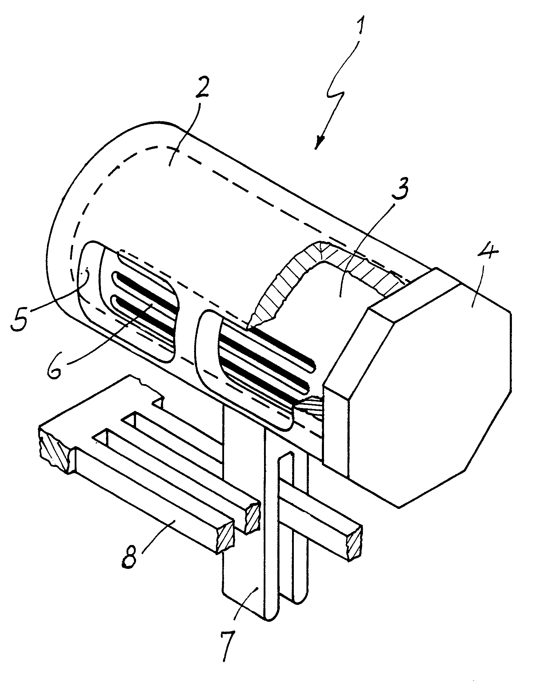

The details of the invention will become apparent from the description of the deodorant composition, and of a containment apparatus adapted for its containment, illustrated in the accompanying drawing, wherein:
the only figure is a perspective view of a containment device for the deodorant composition according to the invention.

The deodorant composition according to the invention is constituted by a gelatine obtained starting from essential oil, pure or possibly diluted with an appropriate solvent in an oil/solvent ratio of 1:1-2.

The solvent is chosen preferably among alcohols and glycols, though any suitable organic solvent can be used.

Said essential oil is mixed with an amount of a suitable thickening agent, which is less than 50% by weight of the deodorant composition.

Said thickening agent is preferably constituted by colloidal silica, of the type for example known by the commercial name of "CAB-O-SIL", produced by the company Cabot Italiana SpA, in Ravenna (ITALY), or "Aerosil", produced by the Degussa Company of Frankfurt, West Germany.

The percentage of colloidal silica can vary according to the degree of hardness of the gelatine which it is desired to obtain and to the type of essential oil employed.

More in particular, the essential oil is contained in the gelatine in a percentage of 60 - 96% and the colloidal silica in a percentage of 4 - 40% by weight. A typical formulation comprises, by way of example, 92% of essence and 8% of colloidal silica. The scenting of the essence can naturally be any (flowers, lavender, pine and the like).

The preparation of the deodorant composition occurs by adding the appropriate amount of colloidal silica to the essential oil under agitation, for example by means of a screw mixer.

Finally a water-free deodorant composition is obtained, containing in small amounts of gelatine large amounts of essential oil. This allows to limit the dimensions of a deodorant package made from the deodorant composition according to the invention, maintaining the same features of emission intensity and duration of the larger ones.

The obtained gelatine is adapted to be placed within a containment device of the type illustrated in the drawing and generally indicated at 1. The device 1, made of plastic material, by molding, is constituted by an outer cylindrical shell 2 open at one end and bearing, rotatably inserted, a container 3 of the deodorant, equally cylindrical in shape. The container 3 defines at one end a head 4 with polygonal profile, intended to remain outside the outer shell 2 so as to allow the angular rotation of said container 3.

The outer shell 2 is provided, in diametrally opposite positions, with respective pairs of openings 5. A plurality of slots 6, longitudinally provided in corresponding diametrally opposite regions of the container 3, is intended to face said openings 5. Finally, the outer shell 2 defines externally a fork

7 extended radially and adapted for the elastic coupling of the device on suitable supports. In particular the fork 7 allows the coupling of the device 1 to the flaps 8 of conventional aeration ports usually provided inside cars.

By rotating the container 3 of the gelatine with respect to the outer shell 2 it is possible to arrange the slots 6 of the former more or less facing the openings 5 of the latter, so as to graduate in the most convenient manner the release of deodorant into the environment and ensuring thereby its long duration.

The following examples illustrate only by way of example possible preparations according to the invention.

EXAMPLE 1

A deodorant composition is prepared mixing 8.8 kg of SHG 680 cologne essential oil (available from CREATIONS ET PARFUMS S.r.l. of Milan, Italy) with 1.2 kg of CAB-O-SIL colloidal silica.

The components are loaded at room temperature in a screw mixer and are mixed until a homogeneous past is obtained. The duration was 5 minutes when standardized screw mixers were used.

The paste obtained is dispensed into containers as described above by means of a screw dispenser.

EXAMPLE 2

The process described above is repeated, mixing 8.6 kg of A 439330 lavender essential oil (available from NARDEN Italiana, of Bologna, Italy) with 1.4 kg of CAB-O-SIL colloidal silica, obtaining a paste.

The above described preparations have a long scent emission duration at sustained intensity in particular of 3-8 weeks as compared to a duration of generally 3 weeks for conventional deodorants.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

Claims

1. Deodorant composition for enclosed spaces, in particular for cars, houses and the like, essentially consisting of a gelatine of essential oil and thickening agent, characterized in that said gelatine comprises a mixture of at least 50% by weight of at least one essential oil with less than 50% of a thickening agent compatible with said oil.

2. Deodorant composition according to claim 1, wherein said thickening agent is constituted by colloidal silica.

3. Deodorant composition according to claim 2, wherein the colloidal silica is chosen among those available under the trade names "CAB-O-SIL" and "Aerosil".

4. Deodorant composition according to any one of the preceding claims, comprising from 60 to 96% by weight of said essential oil and from 40 to 4% of said thickening agent.

5. Deodorant composition according to claim 1 or 2, furthermore comprising a solvent for said essential oil in a weight ratio between oil and solvent of 1:1-2.

6. A process for obtaining a deodorant composition of controllable scent emission intensity according to claim 1, characterized in that at least 50% by weight of at least one essential oil is mixed at room temperature with less than 50% by weight of a thickening agent compatible with said oil until a homogeneous past is obtained.

7. An apparatus for controlling the scent emission of the deodorant composition according to claim 1, characterized in that it comprises an outer cylindrical shell (2) having at least one lateral opening (5), and a container (3) of said deodorant rotatably inserted in said outer shell (2) and having a plurality of longitudinal slots (6) adapted to face, more or less, according to the reciprocal angular position of said outer shell (2) and said container (3), said opening (5) so as to regulate release of said deodorant in enclosed spaces.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-2 871 526  (J.J. BULLOFF)<br>* Column 7, lines 51-58; column 8, lines 1-24 * | 1-4,6 | A 61 L   9/04<br>A 61 L   9/01 |
| Y | | 5,7 | |
| | --- | | |
| X | EP-A-0 105 539  (NAARDEN)<br>* Page 4, lines 22-26; page 5, lines 10-16; examples I-III; claims 1,2,5 * | 1-3 | |
| Y | | 5 | |
| | --- | | |
| Y | FR-A-1 596 401  (DYNACHIM)<br>* Figure 10 * | 7 | |
| | --- | | |
| Y | FR-A-1 500 142  (A.L.F. SERVANT)<br>* Figure 2 * | 7 | |
| | --- | | |
| A | US-A-3 661 838  (F. ENOMOTO)<br>* Column 9, table 1 * | 1-3 | |
| | --- | | |
| A | US-A-3 767 787  (H.A. SEGAL)<br>----- | | |

TECHNICAL FIELDS SEARCHED (Int. Cl.4)

A 61 L   9/04

The present search report has been drawn up for all claims

| Place of search | Date of completion of the search | Examiner |
|---|---|---|
| THE HAGUE | 12-10-1988 | PELTRE CHR. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document